# EUROPÄISCHE PATENTANMELDUNG

(11) **EP 2 116 212 A1**
(43) Veröffentlichungstag der Anmeldung: **11.11.2009**
(21) Anmeldenummer: 08008424.7
(22) Anmeldetag: 05.05.2008
(51) Int. Cl.: A61F 2/44

(54) **Prothese, insbesondere Zwischenwirbelprothese**

(71) Anmelder: Röbling, Christian, 79104 Freiburg (DE); Ackermann, Rolf, 78604 Rietheim-Weilheim (DE)
(72) Erfinder: Röbling, Christian, 79104 Freiburg (DE); Ackermann, Rolf, 78604 Rietheim-Weilheim (DE)
(74) Vertreter: Modrow, Stephanie

(57) **Zusammenfassung**

Die Erfindung betrifft eine Prothese, insbesondere Zwischen-wirbelprothese, mit wenigstens einem Verankerungselement (50,50') zur Verankerung der Prothese in dem benachbarten Knochen, insbesondere dem benachbarten Wirbel, wobei das Verankerungselement (50,50') in der Prothese versenkbar und zumindest teilweise aus der Prothese herausbewegbar angeordnet ist.

## Beschreibung

Die Erfindung betrifft eine Prothese, insbesondere eine Zwischenwirbelprothese gemäß dem Oberbegriff des Patentanspruchs 1.

Bekannt sind Prothesen, insbesondere Zwischenwirbelprothesen mit wenigstens einer Prothesenplatte, welche auf wenigstens einer Seite Verankerungselemente, insbesondere Verankerungszähne, aufweisen, um die Zwischenwirbelprothese zuverlässig im Knochen, insbesondere an den benachbarten Wirbeln zu verankern. Um die Zwischenwirbelprothese in den Zwischenraum zwischen zwei benachbarten Wirbeln nach Entfernen der defekten Bandscheibe einführen zu können, muss der Raum zwischen den beiden benachbarten Wirbeln aufgespreizt werden, um die Zwischenwirbelprothese einführen zu können. Verringert man anschließend die Aufspreizung, stoßen die Verankerungszähne in die benachbarten Wirbel, um die Zwischenwirbelprothese zu fixieren.

Die Aufgabe der Erfindung besteht darin, eine Prothese, insbesondere eine Zwischenwirbelprothese bereitzustellen, welche einfacher zu implantieren ist.

Die Aufgabe wird erfindungsgemäß gelöst durch eine Prothese mit den Merkmalen des Anspruchs 1.

Die erfindungsgemäße Prothese weist wenigstens eine Prothesenplatte auf, bei welcher an der Prothesenplatte wenigstens ein Verankerungselement zur Verankerung der Prothese in dem benachbarten Knochen oder Wirbel angeordnet ist, wobei das Verankerungselement in der Prothese versenkbar und zumindest teilweise aus der Prothese herausbewegbar angeordnet ist. Dadurch wird es ermöglicht, die Prothese mit zunächst versenkten Verankerungselementen in den Knochen oder in den Zwischenraum zwischen zwei benachbarten Wirbeln einzuschieben, wobei der Knochen weniger belastet bzw. der Zwischenraum weniger aufgespreizt werden muss, als es bei fest auf der Prothese angeordneten Verankerungselementen der Fall wäre. Ist die Prothese richtig positioniert, werden die Verankerungselemente aus der Prothese herausbewegt und stoßen somit in den benachbarten Knochen oder die benachbarten Wirbel, um die Zwischenwirbelprothese zu fixieren.

Bevorzugt ist das Verankerungselement in einer Verankerungsausnehmung angeordnet und durch ein Betätigungselement zumindest teilweise aus der Verankerungsausnehmung der Prothese herausbewegbar. Das Betätigungselement kann dabei ebenfalls an der Zwischenwirbelprothese angeordnet oder als separates Werkzeug, welches anschließend aus dem Zwischenwirbelraum entfernt wird, ausgebildet sein.

Vorzugsweise weist das Betätigungselement wenigstens eine Zwangsfläche auf, auf welcher bei Bewegung des Betätigungselementes wenigstens eine Auflagekante des Verankerungselementes läuft, um auf diese Weise die Bewegung des Verankerungselementes oder zumindest eines Teiles davon aus der Aufnahmeausnehmung heraus zu bewirken. Vorzugsweise ist die Zwangsfläche dazu keil-, konus- oder kegelförmig ausgebildet.

Wird lediglich eine Prothese, beispielsweise eine Zwischenwirbelprothese mit nur einer Prothesenplatte zwischen zwei benachbarte Wirbel eingesetzt, ist bei einer besonders bevorzugten Ausführungsform der Erfindung das Verankerungselement durch das Betätigungselement zumindest teilweise aus der Verankerungsausnehmung auf zwei gegenüberliegenden Seitenflächen der Prothese herausbewegbar. Dadurch wird es ermöglicht, die Prothese mit einer Bewegung gleichzeitig an den beiden benachbarten Wirbeln zu fixieren, wodurch das Einsetzen der Prothese weiter vereinfacht wird.

Eine besonders einfache Ausgestaltung von Verankerungselementen ergibt sich, wenn das Verankerungselement U-förmig ausgebildet ist, wobei wenigstens ein freies Ende eines der Schenkel des U-förmigen Verankerungselementes an der Zwangsfläche des Betätigungselementes anliegt, welches auf diese Weise bei Bewegung des Betätigungselementes derart über die Zwangsfläche läuft, dass es aus der Verankerungsausnehmung der Prothese herausgeführt wird.

Gemäß einer besonders bevorzugten Ausführungsform der Erfindung weist das Verankerungselement ein Rastelement auf, welches in der aus der Ausnehmung herausbewegten Position mit einem Rastelement des Betätigungselementes zusammenwirkt, um auf diese Weise zu verhindern, dass nach Einbringen der Zwischenwirbelprothese in den Zwischenwirbelraum und Ausfahren der Verankerungselemente versehentlich die Verankerungselemente in die Verankerungsausnehmung zurückgleiten können und die Gefahr besteht, dass sich die Prothese löst.

Das Rastelement des Verankerungselementes ist vorzugsweise als an einem freien Ende eines der Schenkel des U-förmigen Verankerungselementes angeordneter Rastvorsprung und das Rastelement des Betätigungselementes als im Anschluss an die keil- oder kegelförmige Zwangsfläche angeordnete umlaufende Nut ausgebildet, was eine besonders einfache Rastverbindung ermöglicht.

Gemäß einer bevorzugten Ausnehmungsform der Erfindung ist die Prothese aus PEEK (Polyetheretherketon) gefertigt, welches gute biokompatible Eigenschaften aufweist. Ein besonderer Vorteil dieses Materials liegt darin, dass es sowohl CT(Computertomographie)- als auch MRT(Magnetresonanztomographie)-kompatibel ist und nicht, wie insbesondere verschiedene Metalle, Artefakte in den erstellten Bildern liefert.

Die Erfindung wird anhand der nachfolgenden Figuren ausführlich erläutert.

### Es zeigen

- Fig. 1a: eine perspektivische Ansicht einer Prothesenplatte einer Zwischenwirbelprothese,
- Fig. 1b: die Prothesenplatte gemäß Fig. 1a in einer weiteren perspektivischen Darstellung,
- Fig. 1c: ein Verankerungselement und ein Betätigungselement in perspektivischer Ansicht,
- Fig. 1d: das Verankerungselement gemäß Fig. 1c mit aufgesetztem Betätigungselement und
- Fig. 1e: die erste Prothesenplatte gemäß Fig. 1a mit eingesetztem Verankerungselement gemäß Fig. 1c,
- Fig. 2a: das Verankerungselement und das Betätigungselement gemäß Fig. 1c in einer alternativen Prothese in einer versenkten Position,
- Fig. 2b: das Verankerungselement und das Betätigungselement gemäß Fig. 2a in einer weiteren Seitenansicht,
- Fig. 2c: das Verankerungselement und das Betätigungselement gemäß Fig. 2a in einer herausbewegten Position,
- Fig. 2d: das Verankerungselement und das Betätigungselement gemäß Fig. 2c in einer weiteren Seitenansicht,
- Fig. 3: eine alternative Ausgestaltung eines Verankerungselements,
- Fig 4a: eine weitere alternative Ausgestaltung eines Verankerungselements,
- Fig 4b: eine weitere alternative Ausgestaltung eines Verankerungselements,
- Fig 4c: eine weitere alternative Ausgestaltung eines Verankerungselements,
- Fig 4d: eine weitere alternative Ausgestaltung eines Verankerungselements,
- Fig 4e: eine weitere alternative Ausgestaltung eines Verankerungselements,
- Fig 4f: eine weitere alternative Ausgestaltung eines Verankerungselements und
- Fig 4g: eine weitere alternative Ausgestaltung eines Verankerungselements.

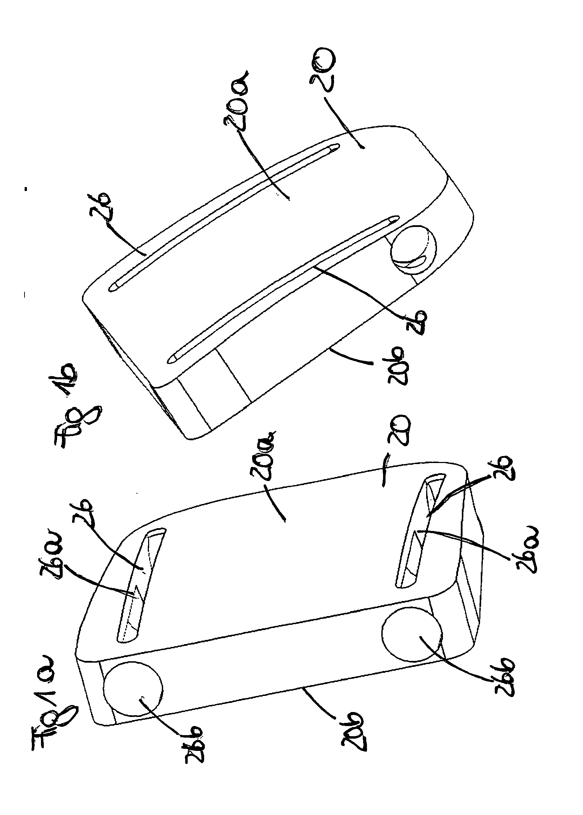

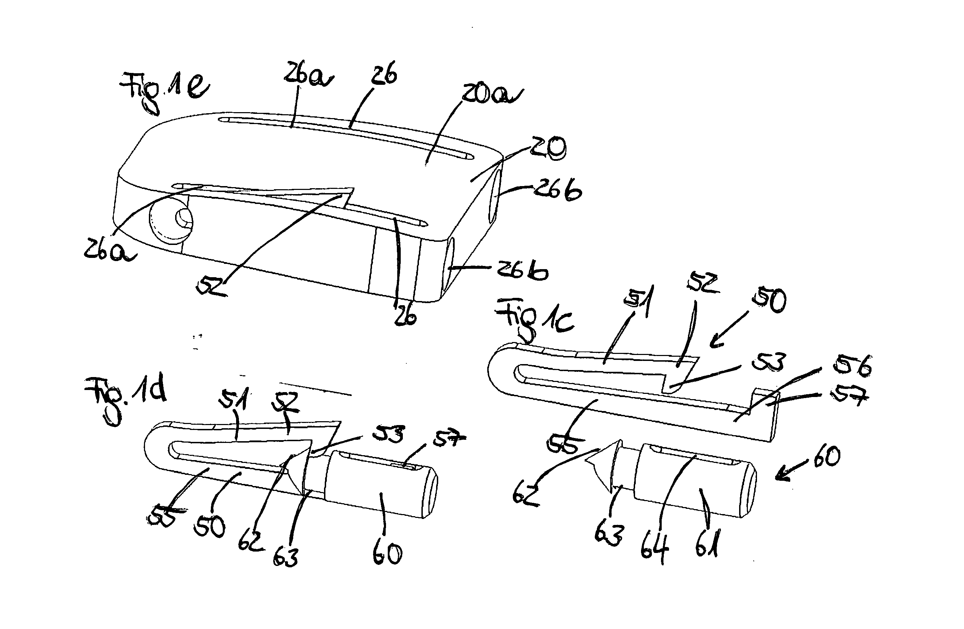

In den Figuren 1a-1e ist ein Verankerungsmechanismus zur Verankerung einer Prothesenplatte 20 in einem benachbarten Wirbel dargestellt. Die Prothesenplatte 20 kann eine vollständige Zwischenwirbelprothese bilden oder Teil einer mehrteiligen Zwischenwirbelprothese sein. In den Figuren 1a und 1b wird eine dem benachbarten Wirbel zugewandte Seite 20a der Prothesenplatte 20 gezeigt. Die gegenüberliegende Seitenfläche 20b liegt, wenn die Prothesenplatte 20 eine einteilige Zwischenwirbelprothese bildet, an dem zweiten benachbarten Wirbel an. Falls die Prothesenplatte 20 Teil einer mehrteiligen Zwischenwirbelprothese ist, kann auf der gegenüberliegenden Seitenfläche 20b beispielsweise eine konkave Ausnehmung oder ein konvexer Vorsprung angeordnet sein, welche mit einem entsprechenden Vorsprung oder einer entsprechenden Ausnehmung eines weiteren Teil der Zwischenwirbelprothese eine Art Kugelgelenk bilden, um auf diese Weise eine flexible und die zu ersetzende Bandscheibe gut simulierende Zwischenwirbelprothese darzustellen.

Die Prothesenplatte 20 weist in ihrer dem benachbarten Wirbel zugewandten Seite 20a zwei parallel zueinander in sagittaler Richtung verlaufende Verankerungsausnehmung 26 auf, in welchen jeweils ein Verankerungselement 50 angeordnet werden kann. Das Verankerungselement 50 ist etwa U-förmig ausgebildet mit einem ersten Schenkel 51 und einem zweiten Schenkel 55, wobei der zweite Schenkel 55 länger ausgebildet ist als der erste Schenkel 51. Der zweite Schenkel 55 weist ein freies Ende 56 auf, an welchem ein nach innen abgewinkelter Haltevorsprung 57 angeordnet ist.

Das Verankerungselement 50 kann mit Hilfe eines Betätigungselementes 60 in der Verankerungsausnehmung 26 versenkt und zumindest teilweise aus dieser herausbewegt werden. Das Betätigungselement 60 weist einen etwa zylindrischen Grundkörper 61 auf, an welchem an einem Ende eine Zwangsfläche 62 angeordnet ist, welche etwa kegelförmig ausgebildet ist. Im Anschluss an die Zwangsfläche 62 weist der Grundkörper 61 eine umlaufende Nut 63 geringeren Durchmessers als der Durchmesser des Grundkörpers 61 auf. In dem Grundkörper 61 ist diametral ein Querschlitz 64 angeordnet. Der Querschlitz 64 ist entlang eines Radius durch die Nut 63 und die Zwangsfläche 62 bis zum durch die Zwangsfläche 62 gebildeten Ende des Betätigungselementes 60 fortgesetzt. Auf diese Weise wird es ermöglicht, das Betätigungselement 60 auf das freie Ende 56 des zweiten Schenkels 55 des Verankerungselementes 50 derart aufzusetzen, dass der Haltevorsprung 57 in den Querschlitz 64 eingreift und das Betätigungselement 60 entlang des freien Endes 56 des zweiten Schenkels 55 des Verankerungselementes 50 über den Fortsatz des Querschlitzes 64 in der Nut 63 und der Zwangsfläche 62 linear geführt bewegbar ist.

Der erste Schenkel 51 des Verankerungselementes 50 weist ein freies Ende 52 auf, an welchem sich nach innen ein Rastvorsprung 53 erstreckt. Ist das Betätigungselement 60 auf dem zweiten Schenkel 55 am weitesten in Richtung des freien Endes 56 des zweiten Schenkels 55 bewegt, liegt der Rastvorsprung 53 vor der Zwangsfläche 62. Wird das Betätigungselement 60 entlang des zweiten Schenkels 55 in Richtung auf den die beiden Schenkel 51, 55 verbindenden Bereich des U-förmigen Verankerungselementes 50 geschoben, bewirkt die keil- oder kegelförmige Zwangsfläche 62 des Betätigungselementes 60, dass der Rastvorsprung 53 mit einer Auflagekante auf ihm entlang laufend von dem zweiten Schenkel 55 wegbewegt und das U-förmige Verankerungselement 50 aufgebogen wird.

Das Verankerungselement 50 wird in einem ersten Teilbereich 26a der Verankerungsausnehmung 26 der ersten Prothesenplatte 20 derart angeordnet, dass die Ebene des U des Verankerungselementes 50 senkrecht zur Seite 20a bzw. 20b verläuft. Das Verankerungselement 50 ist derart dimensioniert, dass es im nicht aufgebogenen Zustand in der Verankerungsausnehmung 26 vollständig eingebettet ist.

Der etwa schlitzförmige und zur Seite 20a offene Teilbereich 26a der Verankerungsausnehmung 26 setzt sich in sagittaler Richtung nach anterior versetzt in einem zylindrischen Teilberich 26b fort, in welchem das Betätigungselement 60 linear verschiebbar geführt angeordnet werden kann. Wird das Betätigungselement 60 entlang des zweiten Schenkels 55 in die erste Prothesenplatte 20 hineingedrückt, bewirkt die Zwangsfläche 62 ein Aufbiegen des ersten Schenkels 51 des Verankerungselementes 50, sodass das freie Ende 52 des ersten Schenkels 51 über die Seite 20a hinaussteht (vgl. Fig. 8e). Diese hervorstehenden Bereiche des Verankerungselementes 50 werden in den benachbarten Wirbelkörper hineingedrückt und bewirken auf diese Weise eine Verankerung der Prothesenplatte 20 an dem benachbarten Wirbel. Wird jedoch die Zwischenwirbelprothese 10 in den Zwischenwirbelraum eingeführt, sind die Verankerungselemente 50 zunächst in der Verankerungsausnehmung 26 versenkt, sodass das Einführen der Zwischenwirbelprothese 10 in den Zwischenwirbelraum vereinfacht wird, da der Zwischenwirbelraum weniger aufgespreizt werden muss.

Die Prothesenplatte 20 ist aus PEEK (Polyetheretherketon) gefertigt, da dieses Material besonders gute biokompatible Eigenschaften hat und zudem sowohl CT(Computertomographie)- als auch MRT(Magnetresonanztomographie)-kompatibel ist. Damit die Prothesenplatte 20 jedoch trotzdem in CT- oder MRT-Bildern erkennbar ist, können das Verankerungselement 50 und/oder das Betätigungselement 60 aus Metall gefertigt sein. Zusätzlich oder alternativ können an der Prothesenplatte auch weitere Röntgenmarker beispielsweise in Form von kleinen Metallkugeln oder Metallflächen an festgelegten Positionen auf der Oberfläche der Prothesenplatte 20 angeordnet werden.

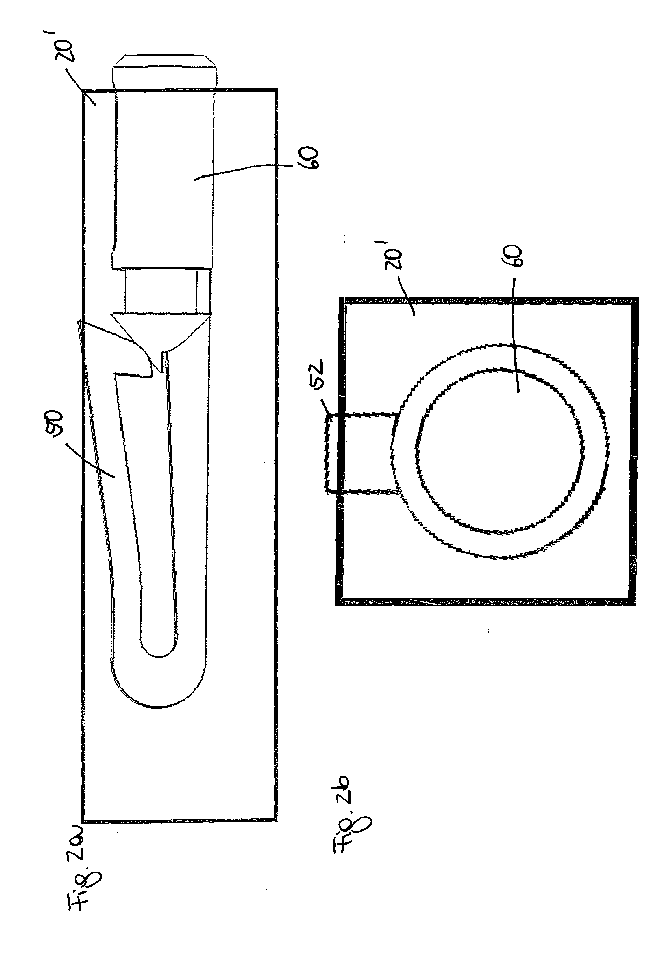

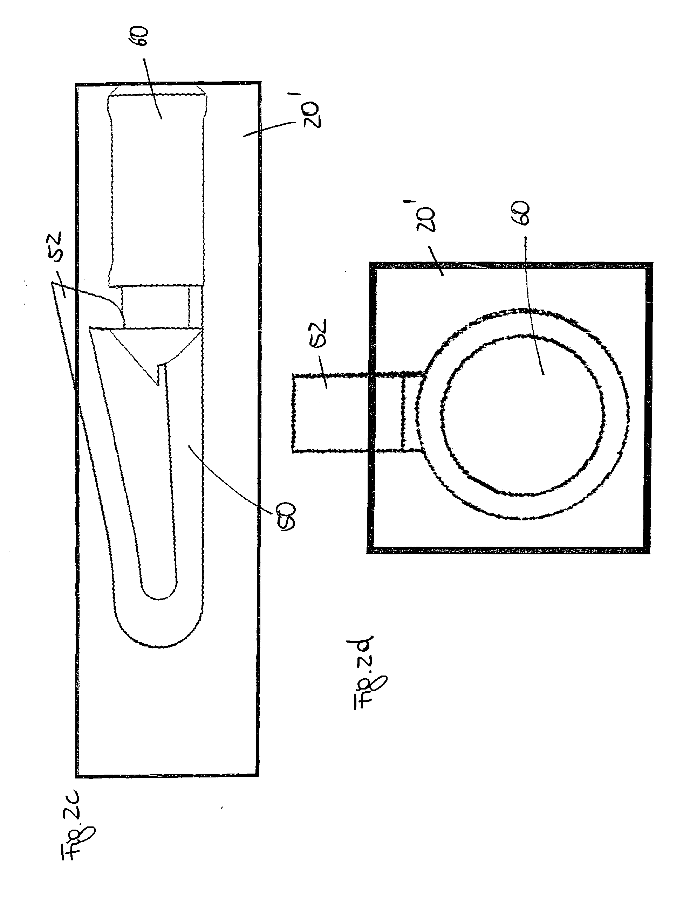

In den Figuren 2a bis 2d ist der Verankerungsmechanismus mit dem Verankerungselement 50 und dem Betätigungselement 60 gemäß den Figuren 1a bis 1d in eine alternative Ausführungsform einer Prothese 20' eingesetzt, welche im wesentlichen als langgestreckter Quader ausgebildet ist, welcher eine zu der Prothesenplatte 20 gemäß den Figuren 1a bis 1d vergleichbare Verankerungsausnehmung aufweist, in welche der Verankerungsmechanismus einsetzbar ist. Die Figuren 2a und 2b zeigen das Verankerungselement 50 in einer eingefahrenen Position, in welcher das das freie Ende 52 des ersten Schenkels 51 nicht oder nur kaum über die Seitenfläche der Prothese 20' hinausragt, so dass die Prothese 20' ohne großen Widerstand in einen Knochen oder in einen Zwischenwirbelraum eingeschoben werden kann. Die Figuren 2c und 2d zeigen das Verankerungselement 50 in einer herausbewegten Position, nachdem das Betätigungselement 60 in die Prothese 20' eingedrückt und der erste Schenkel 51 mit dem freien Ende 52 aufgebogen wurde, so dass sich das freie Ende 52 in den benachbarten Knochen, insbesondere einen benachbarten Wirbel drücken kann, um die Prothese 20' in der entsprechenden Position zu verankern.

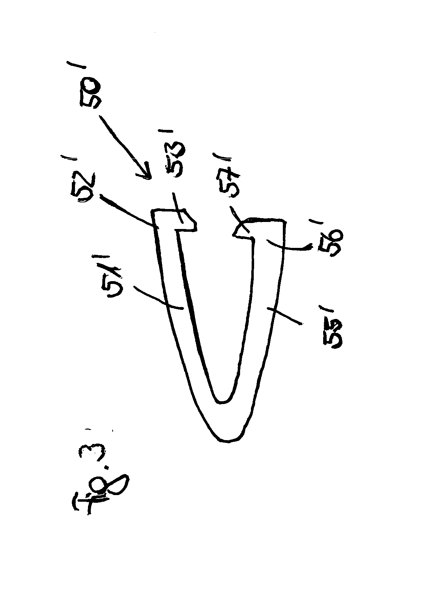

Figur 3 zeigt eine alternative Ausgestaltung eines Verankerungselements 50', welches einer Zwischenwirbelprothese zum Einsatz kommen kann, die lediglich eine Prothesenplatte aufweist. Das Verankerungselement 50' ist etwa U-förmig ausgebildet mit einem ersten Schenkel 51' und einem zweiten Schenkel 55', wobei beide Schenkel 51' und 55' etwa gleich lang ausgebildet sind. Der erste Schenkel 51' des Verankerungselementes 50' weist ein freies Ende 52' auf, an welchem sich nach innen ein Rastvorsprung 53' erstreckt. Auch der zweite Schenkel 55' weist ein freies Ende 56' auf, an welchem sich nach innen ein Rastvorsprung 57' erstreckt.

Die Prothesenplatte weist eine sich in axialer Richtung durch die Prothesenplatte erstreckende Verankerungsausnehmung auf, welche zu beiden gegenüberliegenden Seitenflächen offen ist, so dass, wenn ein Betätigungselement zwischen die beiden freien Enden 52', 56' des Verankerungselemente 50' eingreift, beide Rastvorsprünge 53', 57' an einer entsprechend geformten Zwangsfläche des Betätigungselements mit einer Anlagekante anliegen und beide freien Enden 52', 57' bei Bewegung des Betätigungselements auseinandergespreizt werden, so dass anschließend die freien Enden 52', 57' der ersten Schenkel 51', 55' des Verankerungselements 50' über die Seitenflächen hinausstehen und in die benachbarten Wirbel stoßen. Dabei kann die Prothesenplatte sowohl wie in dem Ausführungsbeispiel gemäß den Figuren 1a bis 1d als auch wie in dem Ausführungsbeispiel gemäß den Figuren 2a bis 2d geformt sein oder auch weitere beliebige Formen je nach Einsatzzweck der Prothese aufweisen.

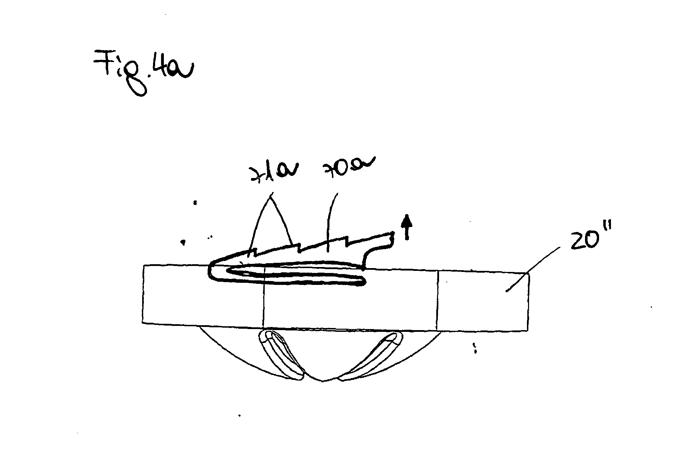

In den Figuren 4a bis 4g sind weitere Ausführungsbeispiele von Verankerungselementen dargestellt. Diese sind in einer Prothesenplatte 20'' angeordnet, welche als Teil einer zweiteiligen Zwischenwirbelprothese ausgebildet ist und auf einer Seite einen konvexen Vorsprung aufweist, der mit einer in einer zweiten Prothesenplatte angeordneten konkaven Ausnehmung zusammenwirkt, um eine Art Kugelgelenk zu bildet. Die Form der Prothesenplatte 20'' kann aber beliebig gewählt werden, insbesondere kann die Prothesenplatte 20'' auch analog der Prothesenplatte 20 gemäß den Figuren 1a bis 1d oder analog der Prothese 20' gemäß der Figuren 2a bis 2d ausgebildet sein. In den Figuren abgebildete Pfeile deuten Bewegungsrichtungen von bewegten Teilen an.

Figur 4a zeigt ein Verankerungselement 70a, welches analog zu dem zuvor beschriebenen Verankerungselement 50 U-förmig ausgebildet ist und auf der nach außen gewandten Seite mehrere Zähne 71a aufweist, um eine bessere Verankerung in dem benachbarten Knochen erreichen zu können.

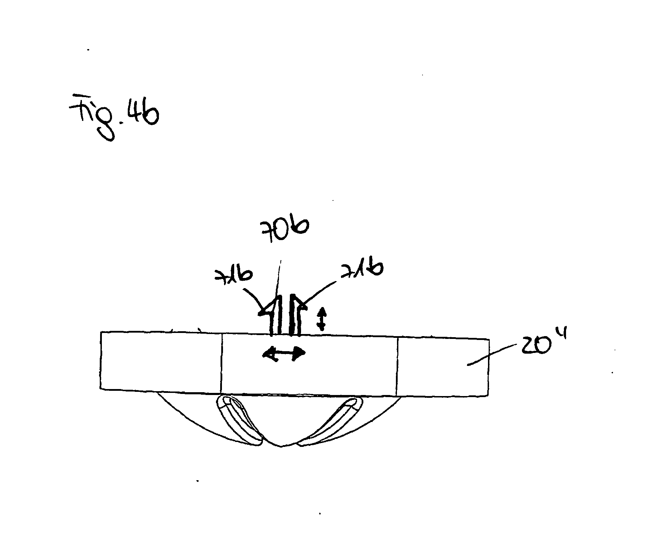

Figur 4b zeigt ein Verankerungselement 70b, welches sowohl senkrecht zur Seitenfläche der Prothesenplatte 20'' aus der Prothesenplatte 20" herausbewegt als auch anschließend quer zu dieser Bewegungsrichtung bewegt werden kann, wobei zusätzliche seitlich angeordnete Vorsprünge 71b eine weitere Verankerungsmöglichkeit quer zur Knochenoberfläche bieten.

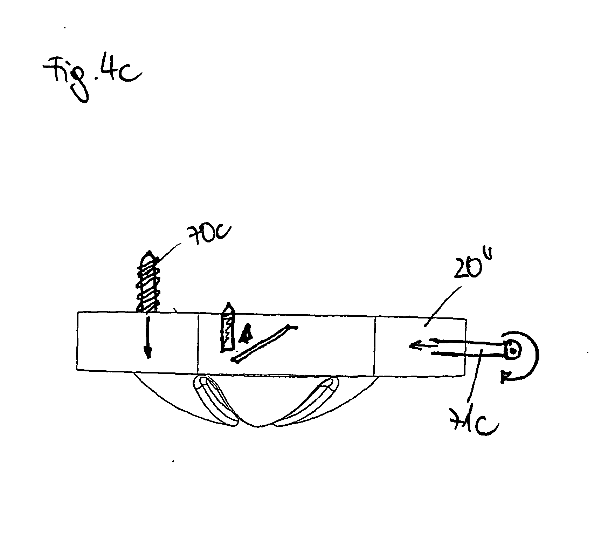

Figur 4c zeigt ein weiteres Ausführungsbeispiel eines Verankerungselement 70c, welches nach Art einer Schraube in Richtung senkrecht zur Oberfläche der Prothesenplatte 20" aus der Prothesenplatte 20'' herausschraubbar ist, beispielsweise durch einen seitlich an der Prothesenplatte 20" angeordneten Drehmechanismus 71c.

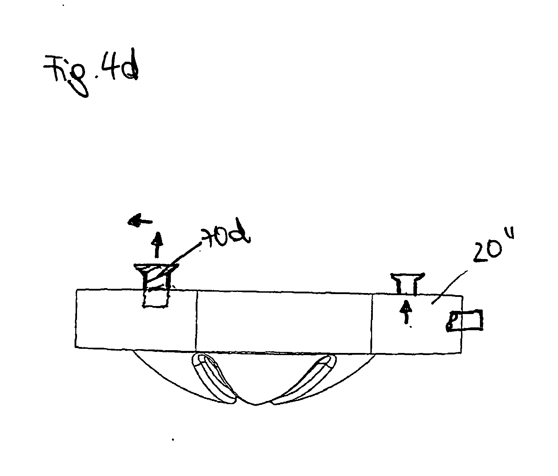

Figur 4d zeigt ein weiteres Ausführungsbeispiel eines Verankerungselements 70d nach Art herausdrückbarer Vorsprünge.

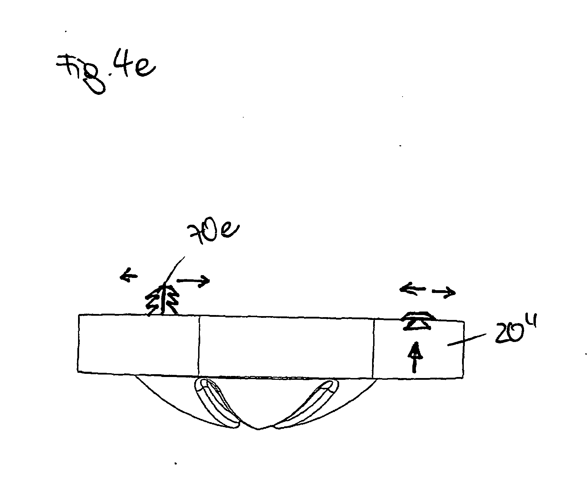

In Figur 4e ist ein weiteres Ausführungsbeispiel eines Verankerungselements 70e dargestellt, welches senkrecht zur Oberfläche der Prothesenplatte 20" herausbewegt wird und etwa die Form und Funktion eines Spreizdübels aufweist.

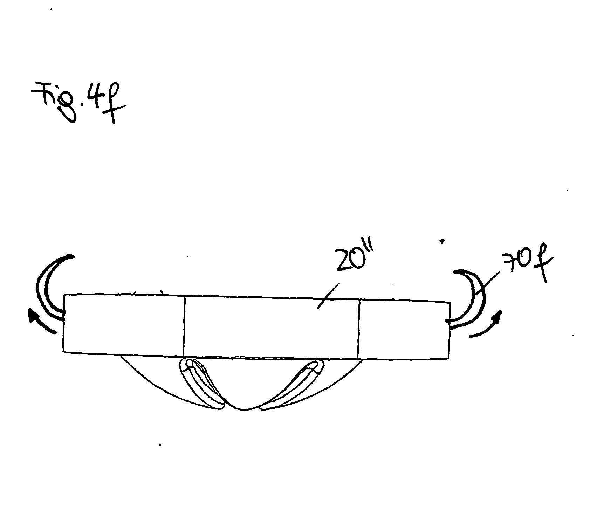

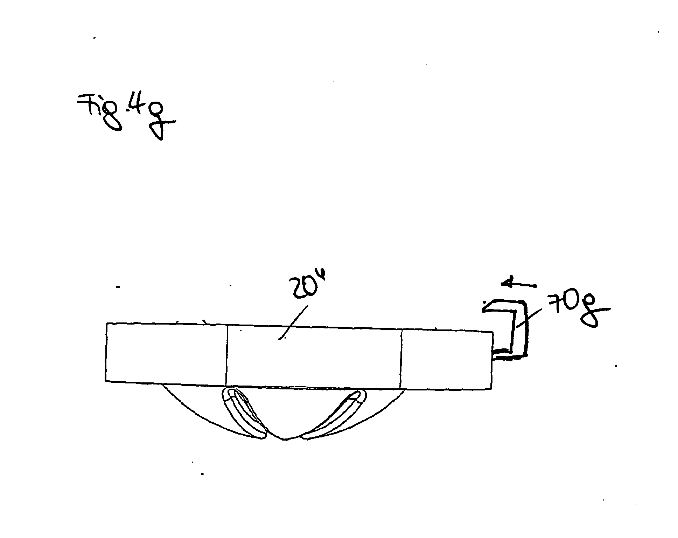

Figur 4f zeigt ein Verankerungselement 70f, welches seitlich aus der Prothesenplatte 20'' herausbewegbar ist und um die Kante der Prothesenplatte 20'' herumgeführt wird, beispielsweise in einer Schwenkbewegung, um eine Verankerung in einer Knochenfläche zu bewirken, welche senkrecht zu der Fläche steht, aus welcher das Verankerungselement 70f herausbewegt wird. Das Verankerungselement 70f ist dabei gebogen ausgebildet, während Figur 4g ein Verankerungselement 70g zeigt, welches ebenfalls seitlich aus der Prothesenplatte herausgeführt wird und als winkliges U ausgebildet ist.

### Bezugszeichenliste

- 20: erste Prothesenplatte
- 20a: Seite
- 20b: Seite
- 26: Verankerungsausnehmung
- 26a: Teilbereich
- 26b: Teilbereich
- 50: Verankerungselement
- 51: erster Schenkel
- 52: freies Ende
- 53: Rastvorsprung
- 55: zweiter Schenkel
- 56: freies Ende
- 57: Haltevorsprung
- 20': Prothese
- 50': Verankerungselement
- 51': erster Schenkel
- 52': freies Ende
- 53': Rastvorsprung
- 55': zweiter Schenkel
- 56': freies Ende
- 57': Rastvorsprung
- 60: Betätigungselement
- 61: Grundkörper
- 62: Zwangsfläche
- 63: Nut
- 64: Querschlitz
- 20'': Prothesenplatte
- 70a: Verankerungselement
- 71a: Zahn
- 70b: Verankerungselement
- 71b: Vorsprung
- 70c: Verankerungselement
- 71c: Drehmechanismus
- 70d: Verankerungselement
- 70e: Verankerungselement
- 70f: Verankerungselement
- 70g: Verankerungselement

## Patentansprüche

1. Prothese, insbesondere Zwischenwirbelprothese, mit wenigstens einem Verankerungselement (50, 50') zur Verankerung der Prothese in dem benachbarten Knochen, insbesondere dem benachbarten Wirbel, angeordnet ist,
**dadurch gekennzeichnet, dass**
das Verankerungselement (50, 50') in der Prothese versenkbar und zumindest teilweise aus der Prothese herausbewegbar angeordnet ist.

2. Prothese nach Anspruch 1,
**dadurch gekennzeichnet, dass**
das Verankerungselement (50, 50') in einer Verankerungsausnehmung (26) angeordnet ist und durch ein Betätigungselement (60) zumindest teilweise aus der Verankerungsausnehmung (26) herausbewegbar ist.

3. Prothese nach Anspruch 2,
**dadurch gekennzeichnet, dass**
das Betätigungselement (60) wenigstens eine Zwangsfläche (62) aufweist, auf welcher bei Bewegung des Betätigungselements (60) wenigstens eine Auflagekante des Verankerungselements (50, 50') läuft.

4. Prothese nach Anspruch 3,
**dadurch gekennzeichnet, dass**
das Betätigungselement (60) eine keil- oder kegelförmige Zwangsfläche (62) aufweist.

5. Prothese nach einem der vorhergehenden Ansprüche,
**dadurch gekennzeichnet, dass** das Verankerungselement (50') durch das Betätigungselement (60) zumindest teilweise aus der Verankerungsausnehmung (26) auf zwei gegenüberliegenden Seitenflächen (20a, 20b) der Prothese herausbewegbar ist.

6. Prothese nach einem der vorhergehenden Ansprüche,
**dadurch gekennzeichnet, dass** das Verankerungselement (50, 50') U-förmig ausgebildet ist, wobei wenigstens ein freies Ende (52, 52') eines der Schenkel (51, 51') des U-förmigen Verankerungselements (50, 50') an der Zwangsfläche (62) des Betätigungselements (60) anliegt.

7. Prothese nach einem der vorhergehenden Ansprüche,
**dadurch gekennzeichnet, dass**
das Verankerungselement (50, 50') ein Rastelement aufweist, welches in der aus der Verankerungsausnehmung (26) herausbewegten Position mit einem Rastelement des Betätigungselements (60) zusammenwirkt.

8. Prothese nach Anspruch 7,
**dadurch gekennzeichnet, dass**
das Rastelement des Verankerungselements (50, 50') als an einem freien Ende (52, 52') eines der Schenkel (51, 51') des U-förmigen Verankerungselements (50, 50') angeordneter Rastvorsprung (53, 53') und das Rastelement des Betätigungselements (60) als im Anschluss an die keil- oder kegelförmige Zwangsfläche (62) angeordnete umlaufende Nut (63) ausgebildet ist.

9. Prothese nach einem der vorhergehenden Ansprüche,
**dadurch gekennzeichnet, dass** die Prothese aus PEEK gefertigt ist.
